# EUROPÄISCHE PATENTSCHRIFT

(11) **EP 0 420 177 B1**
(45) Veröffentlichungstag und Bekanntmachung des Hinweises auf die Patenterteilung: **18.01.1995**
(21) Anmeldenummer: 90118456.4
(22) Anmeldetag: 26.09.1990
(51) Int. Cl.: A61B 5/00, G08C 17/00

(54) **Vorrichtung zur drahtlosen Messung einer lokalen physikalischen Grösse**
Device for wireless measurement of a local physical value
Dispositif pour la mesure sans fil d'une grandeur physique locale

(30) Priorität: 28.09.1989 DE 3932428
(43) Veröffentlichungstag der Anmeldung: 03.04.1991
(73) Patentinhaber: ArguMens Mikrowellenelektronik GmbH, D-4100 Duisburg (DE)
(72) Erfinder: Koster, Norbert H.L., Dr.-Ing., W-5173 Aldenhoven (DE); Wolff, Ingo, Prof. Dr.-Ing., W-5100 Aachen (DE)
(74) Vertreter: Frohwitter, Bernhard, Dipl.-Ing.

(56) Entgegenhaltungen:
- DE-A- 3 219 558
- US-A- 4 075 632

## Beschreibung

Die Erfindung betrifft eine Vorrichtung zur genauen drahtlosen Messung der räumlichen Verteilung lokaler physikalischer Größen, vorzugsweise der Temperaturverteilung, an normalerweise schwer zugänglichen Orten, insbesondere in lebenden Körpern. Die Vorrichtung besteht einerseits aus einer Anzahl miniaturisierter, nahezu vollständig monolithisch integrierbarer Transpondereinrichtungen als Elemente der Meßvorrichtung zur Erfassung der Werte und Meßorte der physikalischen Größen, und andererseits aus einer vom Ort der Messung räumlich entfernt stationierten Einheit zur drahtlosen Energieversorgung und Steuerung der Transpondereinichtungen und zur Auswertung der durch die Transpondereinrichtung vermittelten Informationen. Dabei handelt es sich bei den Transpondern um drahtlos fremdgespeiste, kombinierte Transponder, die sowohl ein individuell verschiedenes Erkennungszeichen abgeben, als auch gleichzeitig lokale physikalische Größen (z.B. die lokale Temperatur) an einem im Normalfall unzugänglichen Ort (z.B. im lebenden Körper) meßtechnisch erfassen und diese Daten drahtlos an eine außerhalb des Meßortes stationierte Auswerteeinheit übertragen. Dadurch, daß die lokalen physikalischen Größen dem Ort des jeweiligen Transponders durch das individuell verschiedene Erkennungszeichen eindeutig zugeordnet werden können, ist es mit Hilfe mehrerer Transponder möglich, die Gradienten dieser physikalischen Größen zu erfassen (z.B. die Temperaturverteilung in einem Gewebestück). Weiterhin kann die Erfindung dazu verwendet werden, verschiedene physikalische Größen (z.B. Druck und Temperatur) gleichzeitig mit Hilfe mehrerer Transponder mit entsprechend geeigneten Meßwertaufnehmern in einem oder aber auch verschiedenen, auch örtlich getrennten Geweben (z.B. mehrere Tiere in einem Stall) zu erfassen.

Die Vorrichtung ist geeignet, mit Hilfe kombinierter Transponder, gleichzeitig, einerseits lokale physikalische Größen, (z.B. die lokale Temperatur an verschiedenen Stellen eines menschlichen Tumorgewebes bei der Anwendung von Hyperthermieverfahren zur Krebstherapie oder die Druckverhältnisse an verschiedenen Orten im Kieferknochen bei Belastung während des Kauens oder die Druckverhältnisse in verschiedenen Bereichen im schlagenden Herzmuskel) genau zu messen und die (z.B. analogen) Meßwerte drahtlos an eine Auswerteeinheit zu übertragen und andererseits ein (z.B. digitales) Erkennungszeichen, welches zur Identifikation des jeweilig aktivierten Transponders von diesem erzeugt wird, zu verarbeiten. Bei Verwendung mehrerer kombinierter Transponder ist es somit möglich, nicht nur die Temperatur in einem einzigen Punkt, sondern die Temperaturverteilung in einem zusammenhängenden oder nicht zusammenhängenden Gewebsteil drahtlos zu messen. Dies ist besonders wichtig in der Tumortherapie mit Hilfe von Hyperthermieverfahren, da dort die genaue Erfassung der Temperatur im Kern des Tumors und in dessen (zumeist besser druchbluteten und damit kühleren) Randbereichen von ausschlaggebender Bedeutung ist. Solange die zu detektierende physikalische Meßgröße in eine zugeordnete elektrische Widerstands- oder Kapazitätsänderung umgesetzt werden kann, ist es möglich, bei Verwendung mehrerer Transponder, z.B. Pulsfrequenz, EKG, Blutdruck, Körpertemperatur eines oder mehrerer Menschen oder Tiere, die sich im Einwirkungsbereich der Steuergeräte befinden, fortwährend drahtlos zu überwachen.

Fremdgespeiste Transponder zur drahtlosen Messung der Temperatur von Objekten oder Lebewesen sind bereits seit geraumer Zeit bekannt und werden - auch in miniaturisierter Form - an einzelnen Exemplaren von Tieren erprobt (DE 32 19 558).
Fremdgespeiste Transponder zur drahtlosen Identifikation von Objekten oder Tieren sind - auch in miniaturisierter Form - bekannt und seit Jahren im Einsatz (Roth, E.: Der Trick mit dem Chip; Stern, Heft Hr. 42, Okt. 87, S. 251).
Die Kombination der Übertragung eines Identifikationssignals einerseits und einer gemessenen physikalischen Größe (Temperatur) andererseits mit Hilfe eines von einem Steuergerät von außen fremdgespeisten (batterielosen), drahtlosen (in ein Hochfrequenzfeld eingebundenen) Transponders ist ebenfalls bekannt (US 4 075 632).

Um die Temperaturverteilung von Tumorgewebe während einer Hyperthermiebehandlung zu überwachen, ist es zur Zeit notwendig, an mehreren Stellen des Gewebes (blutende) Einstiche vorzunehmen und z.B. Lichtleitfasern mit Sensorköpfen in den Patienten einzuführen. Nach der Behandlung durch den Therapeuten müssen die Glasfasern wieder aus den Wunden herausgezogen werden, da sie einerseits mit dem Steuergerät fest verbunden sind und andererseits sehr empfindlich sind und leicht abbrechen. Bei der nächsten Therapiesitzung müssen die nun bereits vernarbten Wunden erneut durch Einstiche eröffnet werden und die Lichtleitfasern wieder in das Tumorgewebe eingebracht werden. Dieser Vorgang wiederholt sich mit jeder Hyperthermiesitzung und führt zu einer sehr starken Belastung des darüberliegenden, meist noch gesunden Gewebes.
Eine Vorrichtung, basierend auf dem in der US 40 75 632 dargestellten Verfahren, ist nicht besser geeignet; denn es würde bedeuten, daß pro Meßstelle eine einige Zentimeter lange Dipol-Antenne durch die Haut nach außen gebracht und dort verbleiben müßte. Somit würden bei z.B. zehn Meßstellen zwanzig feine Drähte, die nicht abknicken dürften, an der Meßstelle auf Dauer, eventuell lebenslang, verbleiben müssen. Abgesehen von der unzumutbaren psychischen Belastung für den Patienten, ist dieses Verfahren wegen der damit verbundenen ständigen Infektionsgefahr (Wundkanal entlang der Drähte) ungeeignet. Weiterhin wird in dem Verfahren nach der US 40 75 632 der Arbeitspunkt des Transponders durch einen in dem Transponder eingebauten Spannungsregler sichergestellt, der die zuviel eingestrahlte Energie in Verlustwärme umwandelt. Damit ist aber eine Miniaturisierung oder gar monolithische Integration ausgeschlossen, da eine räumliche Nähe von Temperatursensoren einerseits und zum Betrieb der Transponder erforderliche wärmeerzeugende Stabilisationsschaltungen andererseits eine genaue Messung der Temperaturverteilung nicht zulassen.

Der Erfindung liegt die Aufgabe zugrunde, die lokale physikalische Größe mit wesentlich verringerter Rückwirkung des Transponders auf den schwer zugänglichen Meßort, insbesondere auf den dort herrschenden Meßwert, zu bestimmen.
Diese Aufgabe wird bei einer Meßvorrichtung mit den Merkmalen des Oberbegriffs des Patentanspruchs 1, wie sie aus der DE-A-32 19 558 bekannt ist, durch die kennzeichnenden Merkmale des Patentanspruchs 1 gelöst.
Zweckmäßige Weiterbildungen der Erfindung gehen aus den Unteransprüchen hervor.

Die Erfindung eröffnet die Möglichkeit, gleichzeitig mehrere lokale physikalische Größen im Inneren eines menschlichen oder tierischen, lebenden Gewebes (z.B. Tumor) oder anderen unzugänglichen Orten zu messen und diese gemessenen Größen durch ein Erkennungszeichen dem Ort der Messung zuzuordnen.

Hierzu wird die von der jeweils zu messenden physikalischen Größe abhängige Schwingfrequenz eines Generators gemessen, wobei der Generator zusätzlich ein individuelles Erkennungszeichen in Form eines digitalen Wortes erzeugt. Dies geschieht so, daß der Generator an einen Resonanzkreis über eine Gleichrichterschaltung angekoppelt wird und einem Hochfrequenzfeld, dessen Stärke regelbar ist, Energie entzieht. Sobald die dem Feld entzogene Energie ausreicht, um den Generator zu betreiben, beginnt dieser zyklisch nacheinander, sowohl im Takt eines Digitalwortes als auch - zur einfacheren meßtechnischen Auswertung - für einen bestimmten Zeitraum ohne Taktung, eine kontinuierliche Schwingung, deren jeweilige Frequenz der jeweils zu messenden physikalischen Größe eindeutig zugeordnet ist, zu erzeugen. Um die Abhängigkeit dieser so erzeugten Frequenz von anderen Größen außer der zu messenden physikalischen Größe - also insbesondere der durch die am Ort des kombinierten Transponders autretenden nicht vorhersagbaren Stärke des speisenden elektromagnetischen Feldes - zu eliminieren, ist der Transponder so konstruiert, daß er einen von der Auswerteeinheit von außen drahtlos zu erkennenden Arbeitspunkt besitzt, in den er durch Variation der Intensität des speisenden hochfrequenten Feldes durch das Steuergerät hineingebracht und dort geeicht und betrieben werden kann. Das von dem Transponder erzeugte Signal wird vom Steuergerät bzw. der Auswerteeinheit dadurch detektiert, daß der Transponder die zur Erzeugung seiner Signale notwendige Energie im Takt dieser Signale mit Hilfe eines auf die Frequenz des speisenden hochfrequenten Feldes abgestimmten Resonanzkreises dem Feld entzieht. Dadurch wird eine Amplitudenmodulation (Funkfelddämpfung) des speisenden Feldes bewirkt, die von der Auswerteeinheit registriert, demoduliert und verarbeitet wird.

Der kombinierte Transponder eignet sich nicht nur für die oben exemplarisch erwähnten Anwendungsfälle, sondern - abhängig von der Verfügbarkeit von Meßwertwandlern, die eine zu messende physikalische Größe in ein geeignetes elektrisch zu verarbeitendes Signal umwandeln - für alle anderen Meßaufgaben an schwer zugänglichen oder beweglichen Orten, bei denen eine strenge Unterscheidung und Zuordnung von einer Vielzahl gleichartiger Signale notwendig und unvermeidbar ist.

Die mit der Erfindung erzielten Vorteile bestehen insbesondere darin, daß eine lokale Messung mit strenger Zuordnung zum Ort der jeweiligen Messung einer physikalischen Größe über nahezu unbegrenzte Zeitbereiche an unzugänglichen Orten beliebig oft durchgeführt werden kann, ohne daß der implantierte kombinierte Transponder gewartet werden muß, oder Energiequellen (Batterien) ausgetauscht werden müssen. Durch die exakte Zuordnung der gemessenen lokalen physikalischen Größe lassen sich bei Verwendung mehrerer, prinzipiell gleichartig aufgebauter aber mit jeweils einem anderen Erkennungszeichen und eventuell anderen Meßwertwandlern ausgestatteter Transponder entweder ganze Felder einer physikalischen Größe oder Felder verschiedener physikalischer Größen an Orten messen, die im Einwirkungsbereich der Steuergeräte liegen. Zusätzlich ist von Bedeutung, daß die Transponder in Mikroform aufgebaut werden können und für alle Zeiten im lebenden Körper verbleiben können. Somit stehen bei einer eventuell später notwendigen Nachmessung selbst nach langer Zeit die Transponder voll einsatzfähig zur Verfügung. Durch die Eigenschaft der Transponder, einen drahtlos erkennbaren reproduzierbaren Arbeitspunkt zu besitzen, ist eine sehr genaue analoge Messung von physikalischen Größen möglich, was mit bisher zur Verfügung stehenden Meßverfahren unter den genannten Randbedingungen nicht möglich ist.

Es zeigen
- Fig. 1: einen Transponder gemäß einem Ausführungsbeispiel der Erfindung;
- Fig. 2: ein Zeitdiagramm der Stromaufnahme des Signalgenerators in dem Transponder nach Fig. 1;
- Fig. 3: eine zur Arbeitspunktbestimmung herangezogene Kennlinie der von dem Signalgenerator in dem Transponder nach Fig. 1 erzeugten Schwingfrequenz in Abhängigkeit von der angelegten Betriebsspannung; und
- Fig. 4: mehrere Transponder nach Fig. 1 während ihres Betriebes durch eine Steuer- und Auswerteeinheit gemäß der Erfindung.

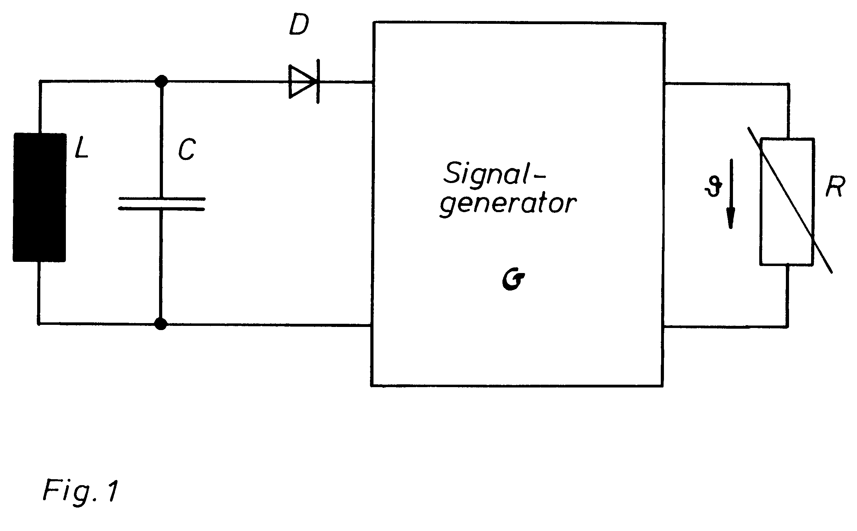

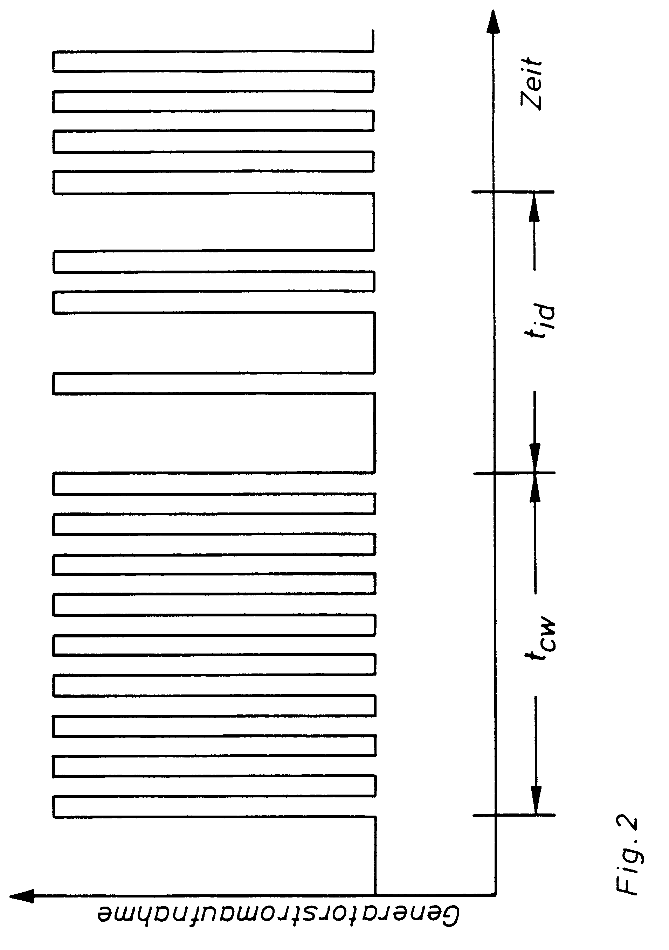

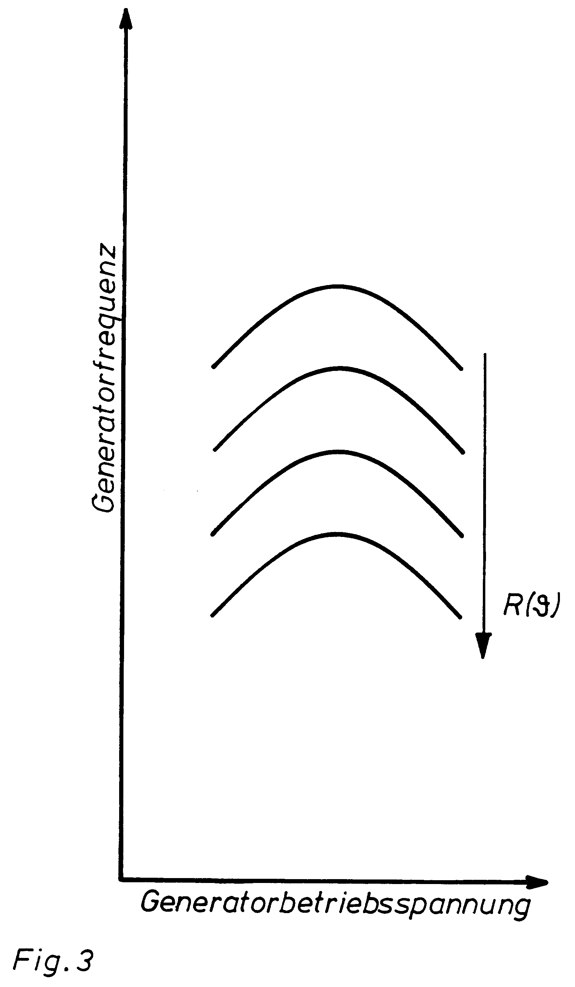

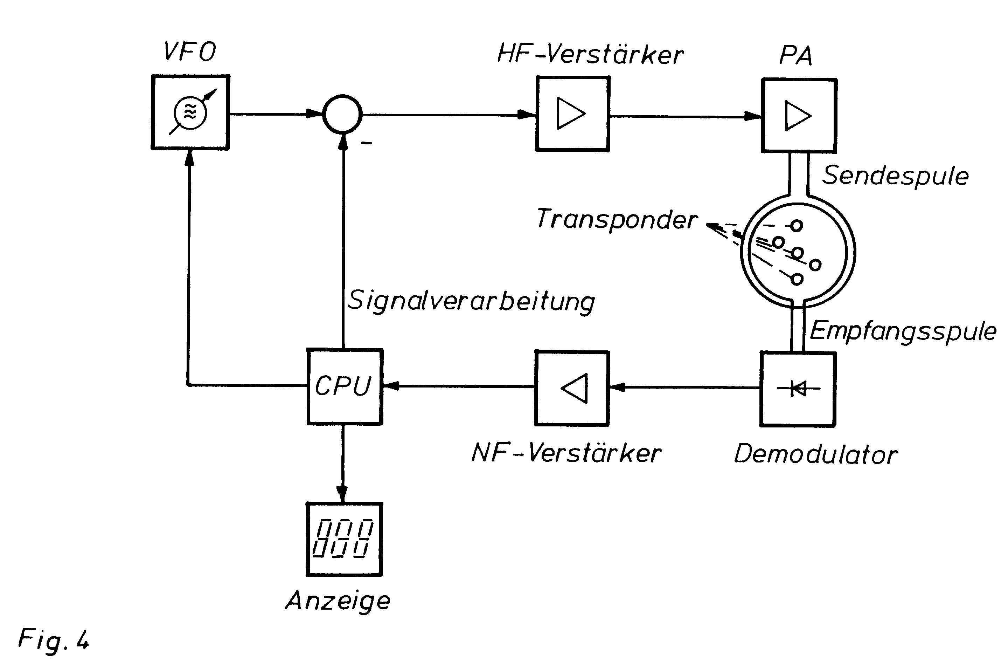

Ein Ausführungsbeispiel der Schaltung eines kombinierten Transponders ist in Fig. 1 gezeigt. Ein Schwingkreis besteht aus einer Spule L und einem Kondensator C und ist auf eine bestimmte Resonanzfrequenz abgestimmt. Wird der Transponder in ein elektromagnetisches Wechselfeld mit der gleichen Frequenz wie die Resonanzfrequenz des Schwingkreises gebracht, so entsteht in diesem Schwingkreis eine hochfrequente Wechselspannung, die von der Diode D gleichgerichtet wird und den Signalgenerator G mit der zum Betrieb erforderlichen Spannung versorgt. Sobald das speisende elektromagnetische Wechselfeld genügend Energie liefert um den Transponder zu betreiben, erzeugt der Signalgenerator G in diesem Transponder im zyklischen Wechsel über den Zeitraum t_{cw} in Fig. 2 eine rechteck- oder sinusförmige Schwingung mit konstanter Amplitude und über den Zeitraum t_{id} in Fig. 2 eine rechteck- oder sinusförmige Schwingung, deren Amplitude im Rhythmus eines Digitalwortes (z.B. I0II0IIII) noch zusätzlich getaktet wird; danach folgt wieder über den Zeitraum t_{cw} eine konstante Amplitude usw. In Fig. 2 ist ein Ausschnitt aus diesem Zyklus zur Veranschaulichung dargestellt. Die Frequenz der Schwingung wird im Falle der Temperaturmessung durch einen Widerstand R, dessen Widerstandswert von der Temperatur abhängt, bestimmt. Andererseits hängt die Frequenz der Schwingung unerwünschterweise auch von der am Generator G anliegenden Betriebsspannung ab. Um diesen Einfluß zu eliminieren, ist der Generator G so beschaffen, daß bei konstanter Temperatur und somit kontantem Widerstandswert R die erzeugte Schwingungsfrequenz zunächst mit wachsender Betriebsspannung ansteigt und nach Erreichen eines Maximums wieder abfällt. Der sich so ergebende Sattelpunkt in der Kennlinie ist der von außen durch die Auswerteeinheit erkennbare Arbeitspunkt des Transponders. Dieser Arbeitspunkt verschiebt sich nur noch in Abhängigkeit von der Temperatur, d.h. also vom Widerstandswert R. In Fig. 3 ist die Kennlinie der von dem Generator G in dem Transponder erzeugten Frequenz der Schwingung in Abhängigkeit von der angelegten Betriebsspannung dargestellt. Der kombinierte Transponder wird in diesem Arbeitspunkt geeicht, sodaß ein reproduzierbarer Zusammenhang zwischen der erzeugten Signalfrequenz im Arbeitspunkt und der zu messenden Temperatur hergestellt werden kann. Durch den Betrieb des Generators G im Transponder wird dem speisenden Hochfrequenzfeld Energie entzogen, was durch eine einfache Ankoppelspule detektiert werden kann. Dabei ist die Funkfelddämpfung durch den Transponder in etwa proportional zum Betriebsstrom des in dem Transponder befindlichen Signalgenerators. Der Betriebsstrom wiederum ist direkt mit den erzeugten Signalen verknüpft. So wird also das vom Steuergerät erzeugte elektromagnetische Feld während der Zeit t_{cw} mit der temperaturabhängigen kontinuierlichen Signalfrequenz des Generators G in dem kombinierten Transponder bedämpft; während des Zeitraumes t_{id} erfolgt die Bedämpfung noch zusätzlich durch ein überlagertes Erkennungszeichen.
Mehrere kombinierte Transponder mit jeweils der in Fig. 1 gezeigten Schaltung, aber zuvor unterschiedlich einprogrammierten Erkennungszeichen werden mit Hilfe der in Fig. 4 gezeigten Steuer- und Auswerteeinheit betrieben. Ein Hochfrequenzgenerator (VFO), dessen Frequenz variabel ist, erzeugt ein Hochfrequenzsignal, dessen Leistung über eine Regelschaltung einem Sendeverstärker (PA) zugeführt wird; dieser wiederum speist eine Sendespule. Das Hochfrequenzsignal der Sendespule induziert in dem Schwingkreis desjenigen Transponders, dessen Resonanzfrequenz mit der Frequenz des VFO übereinstimmt, eine hochfrequente Spannung. Sobald diese Spannung ausreicht, um den durch die VFO-Frequenz ausgewählten Transponder zu betreiben, bedämpft dieser das durch die Sendespule aufgebaute Feld im Rhythmus der Meß- und Identifikationssignale. Diese Funkfelddämpfung wird mit Hilfe einer Empfangsspule, eines Demodulators und Verstärkers demoduliert und einer Signalerkennungs- und Signalverarbeitungseinheit (CPU) zugeführt. Mit Hilfe einer Regelschleife wird nun die Sendeleistung so variiert, daß der angesprochene Transponder im Arbeitspunkt betrieben wird (maximale Signalfrequenz). Nun ist die Signalfrequenz der zu messenden physikalischen Größe eindeutig zugeordnet, der Wert wird in der CPU berechnet und zur Anzeige gebracht. Danach wird die Identifikation des durch die spezielle Wahl der Frequenz des speisenden Wechselfeldes angesprochenen Transponders angezeigt. Nun variiert die CPU die Frequenz des VFO, bis der nächste Transponder wegen des auf eine andere Resonanzfrequenz abgestimmten Schwingkreises angesprochen werden kann. Es erfolgen nun die gleichen Signalverarbeitungsabläufe wie bei dem ersten angesprochenen Transponder. So werden nacheinander alle im Hochfrequenzfeld befindlichen Transponder angesprochen und somit die Verteilung von Temperaturen über einen vorgegebenen Raumbereich erfaßt.

## Patentansprüche

1. Vorrichtung zur drahtlosen Messung einer lokalen physikalischen Größe an einem schwer zugänglichen Ort
- mit einem Hochfrequenzgenerator (VFO), in dessen abgestrahltem hochfrequenten elektromagnetischen Feld eine Transpondereinrichtung (Abb. 1) an dem schwer zugänglichen Ort angeordnet ist, die in Verbindung mit einem Signalgenerator ein niederfrequentes Signal mit einer von der lokalen physikalischen Größe abhängigen Frequenz erzeugt,
- wobei die Transpondereinrichtung ihre Betriebsenergie dem elektromagnetischen Feld über ein Empfangselement mit nachgeschaltetem Gleichrichter entzieht und das elektromagnetische Feld mit dem vom Signalgenerator erzeugten niederfrequenten Signal moduliert,
- und mit einer Empfangseinrichtung für das modulierte elektromagnetische Feld sowie einer Auswerteeinrichtung (CPU), die aus der Frequenz des Signalgenerators dessen lokale Umgebungsgröße ermittelt,
- wobei die Transpondereinrichtung ein miniaturisierter Transponder ist,
- und als Empfangselement für das elektromagnetische Feld in dem Transponder ein Schwingkreis (LC) angeordnet ist,
- und der Signalgenerator (G,R) einen durch die Auswerteeinrichtung (CPU) drahtlos zu erkennenden Arbeitspunkt besitzt,
- und ein mit der Auswerteeinrichtung (CPU) und dem Hochfrequenzgenerator (VFO) verbundener Regelkreis die Frequenz des Hochfrequenzgenerators (VFO) auf die Frequenz des Schwingkreises (LC) des Transponders abstimmt,
**dadurch gekennzeichnet**,
daß der Signalgenerator (G,R) so beschaffen ist, daß die erzeugte Schwingungsfrequenz bei konstanter lokaler Umgebungsgröße mit wachsender Betriebsspannung ansteigt und nach Erreichen eines Maximalwertes wieder abfällt,
daß dieser Regelkreis anschließend die abgestrahlte Hochfrequenzleistung so variiert, daß der Transponder in einem Arbeitspunkt mit maximaler Frequenz betrieben wird,
daß die Auswerteeinrichtung (CPU) die lokale Umgebungsgröße aus einer Verschiebung dieser Maximalfrequenz ermittelt
und, daß der Transponder ein zusätzliches, dem niederfrequenten Signal überlagertes Erkennungszeichen erzeugt.

2. Vorrichtung nach Anspruch 1, dadurch gekennzeichnet, daß der schwer zugängliche Ort lebendes Gewebe und der miniaturisierte Transponder in das Gewebe inplantierbar ist.

3. Vorrichtung nach Anspruch 1 oder 2, dadurch gekennzeichnet, daß mehreren Transpondern vor dem Betrieb unterschiedliche Erkennungszeichen einprogrammiert wurden.

4. Vorrichtung nach Anspruch 3, dadurch gekennzeichnet, daß die Auswerteeinrichtung (CPU) aus dem überlagerten Erkennungszeichen den Ort des Transponders ermittelt.

5. Vorrichtung nach einem der vorhergehenden Ansprüche, dadurch gekennzeichnet, daß das überlagerte Erkennungszeichen digital, d.h. eine digitale Modulation des niederfrequenten Meßsignals ist.

6. Vorrichtung nach einem der vorhergehenden Ansprüche, dadurch gekennzeichnet, daß in dem hochfrequenten elektromagnetischen Feld mehrere individuell programmierte Transponder an verschiedenen schwer zugänglichen Orten mit jeweils einem Empfangselement (LC), einem Signalgenerator mit temperaturabhängigem Element (R) und einem Erkennungszeichen angeordnet sind.

7. Vorrichtung nach einem der vorhergehenden Ansprüche, dadurch gekennzeichnet, daß die lokale physikalische Größe die Temperatur ist.

8. Vorrichtung nach Anspruch 4, dadurch gekennzeichnet, daß eine örtliche Verteilung einer lokalen physikalischen Größe, insbesondere eine Temperaturverteilung, bestimmt wird.

9. Vorrichtung nach Anspruch 4, dadurch gekennzeichnet, daß die Transponder verschiedenartige physikalische Messgrößen, z. B. Druck und Temperatur, an den identifizierbaren Messpunkten erfassen.

## Claims

1. Device for the non-wired measurement of a local physical quantity at a location which exhibits difficult access
- having a radio frequency generator (VFO) in the emitted radio frequency electromagnetic field of which a transponder device (Fig. 1) is disposed at the location exhibiting difficult access, which device, in conjunction with a signal generator, generates an audio frequency signal having a fre-quency dependent upon the local physical quantity,
- in which the transponder device extracts its operating energy from the electromagnetic field via a reception element with a downstream rectifier and modulates the electromagnetic field with the audio frequency signal generated by the signal generator,
- and having a reception device for the modulated electromagnetic field as well as an evaluating device (CPU) which determines from the frequency of the signal generator, its local environmental quantity,
- in which the transponder device is a miniaturized transponder,
- and an oscillator circuit (LC) is disposed in the transponder as reception element for the electromagnetic field,
- and the signal generator (G,R) possesses a working point which is to be identified in non-wired fashion by the evaluating unit (CPU),
- and a control circuit connected to the evaluating device (CPU) and the radio frequency generator (VFO) tunes the frequency of the radio frequency generator (VFO) to the frequency of the oscillator circuit (LC) of the transponder.
characterized in that
the signal generator (G,R) is of such a nature that the generated oscillation frequency increases, at constant local environmental quantity, as the operating voltage increases and, after reaching a maximum value, falls again,
in that this control circuit subsequently varies the emitted radio frequency power, so that the transponder is operated at a working point with maximum frequency, in that the evaluating device (CPU) determines the local environmental quantity from a displacement of this maximum frequency
and in that the transponder generates an additional identification symbol superposed on the audio frequency signal.

2. Device according to Claim 1, characterized in that the location exhibiting difficult access is living tissue and the miniaturized transponder is implantable into the tissue.

3. Device according to Claim 1 or 2, characterized in that, before operation, differing identification symbols were programmed into a plurality of transponders.

4. Device according to Claim 3, characterized in that the evaluating device (CPU) determines the location of the transponder from the superposed identification symbol.

5. Device according to one of the preceding claims, characterized in that the superposed identification symbol is digital, i.e. is a digital modulation of the audio frequency measurement signal.

6. Device according to one of the preceding claims, characterized in that in the radio frequency electromagnetic field a plurality of individually programmed transponders are disposed at various locations exhibiting difficult access, with in each case a reception element (LC), a signal generator with a temperature-dependent element (R) and an identification symbol.

7. Device according to one of the preceding claims, characterized in that the local physical quantity is the temperature.

8. Device according to Claim 4, characterized in that a spatial distribution of a local physical quantity, especially a temperature distribution, is determined.

9. Device according to Claim 4, characterized in that the transponders pick up physical parameters of differing types e.g. pressure and temperature, at the identifiable measurement points.

## Revendications

1. Dispositif pour la mesure, sans fil, d'une grandeur physique locale à un endroit difficilement accessible
- avec un générateur haute fréquence (VFO), dans le champ électromagnétique haute fréquence rayonnant duquel est disposé, à l'endroit difficilement accessible, un appareil transpondeur (figure 1) qui produit, en liaison avec un générateur de signal un signal basse fréquence ayant une fréquence dépendante de la grandeur physique locale,
- l'appareil transpondeur tirant son énergie de fonctionnement du champ électromagnétique par l'intermédiaire d'un élément de réception ayant un redresseur de courant monté en aval et modulant le champ électromagnétique avec le signal basse fréquence produit par le générateur de signal,
- et avec un dispositif de réception pour le champ électromagnétique modulé ainsi qu'un dispositif d'évaluation (CPU) qui détermine à partir de la fréquence du générateur de signal sa grandeur locale environnante,
- l'appareil transpondeur étant un transpondeur miniaturisé,
- et un circuit oscillant (LC) étant agencé dans le transpondeur en tant qu'élément de réception pour le champ électromagnétique,
- et le générateur de signal (G, R) possédant un point de fonctionnement à identifier sans fil grâce au dispositif d'évaluation (CPU),
- et une boucle d'asservissement, reliée au dispositif d'évaluation (CPU) et au générateur haute fréquence (VFO), modulant la fréquence du générateur haute fréquence (VFO) sur la fréquence du circuit oscillant (LC) du transpondeur,
caractérisé en ce que
le générateur de signal (G, R) est alimenté de telle sorte que la fréquence d'oscillation produite augmente avec une tension de fonctionnement croissante pour une grandeur environnante locale constante et, après avoir atteint une valeur maximale, diminue de nouveau,
cette boucle d'asservissement varie suivant la puissance haute fréquence rayonnante de telle sorte que le transpondeur est amené, pour une fréquence maximale, à un point de fonctionnement,
le dispositif d'évaluation (CPU) détermine la grandeur environnante locale à partir d'un décalage de cette fréquence maximale
et le transpondeur produit un signe d'identification supplémentaire, superposé au signal basse fréquence.

2. Dispositif selon la revendication 1, caractérisé en ce que l'endroit difficilement accessible est un tissu vivant et le transpondeur miniaturisé peut être implanté dans le tissu.

3. Dispositif selon la revendication 1 ou 2, caractérisé en ce que des signes d'identification différents ont été programmés dans plusieurs transpondeurs, avant la mise en service.

4. Dispositif selon la revendication 3, caractérisé en ce que le dispositif d'évaluation (CPU) détermine l'endroit du transpondeur à partir du signe d'identification superposé.

5. Dispositif selon l'une des revendications précédentes, caractérisé en ce que le signe d'identification superposé est numérique, c'est-à-dire est une modulation numérique du signal de mesure basse fréquence.

6. Dispositif selon l'une des revendications précédentes, caractérisé en ce que plusieurs transpondeurs programmés individuellement sont disposés dans le champ électromagnétique haute fréquence, en différents endroits difficilement accessibles, avec chacun un élément de réception (LC), un générateur de signal, avec un élément (R) dépendant de la température, et un signe d'identification.

7. Dispositif selon l'une des revendications précédentes, caractérisé en ce que la grandeur physique locale est la température.

8. Dispositif selon la revendication 4, caractérisé en ce que l'on détermine une répartition locale d'une grandeur physique locale, notamment une répartition de la température.

9. Dispositif selon la revendication 4, caractérisé en ce que les transpondeurs effectuent l'acquisition de grandeurs mesurées physiques de différents types, par exemple la pression et la température, en différents points de mesure qui peuvent être identifiés.
